Europäisches Patentamt

European Patent Office

Office européen des brevets

(11) Veröffentlichungsnummer: **0 335 266**

**A1**

(12) # EUROPÄISCHE PATENTANMELDUNG

(21) Anmeldenummer: 89105233.4

(22) Anmeldetag: 23.03.89

(51) Int. Cl.⁴: **A61N 1/08 , A61N 1/18**

(30) Priorität: 31.03.88 DE 3811140

(43) Veröffentlichungstag der Anmeldung:
04.10.89 Patentblatt 89/40

(84) Benannte Vertragsstaaten:
**AT BE CH DE ES FR GB IT LI LU NL SE**

(71) Anmelder: **HENNEBERG & BRUNNER**
**Entwicklungsbüro Goldbachstrasse 3**
**D-6991 Igersheim(DE)**

Anmelder: **CARLE GMBH + CO. KG**
**medizinische Geräte, Einrichtungen und**
**Präparate Beim Braunstall 4**
**D-6990 Bad Mergentheim(DE)**

(72) Erfinder: **Brunner, Elmar**
**Goldbachstrasse 3**
**D-6991 Igersheim(DE)**
Erfinder: **Henneberg, Fred**
**Goldbachstrasse 3**
**D-6991 Igersheim(DE)**

(74) Vertreter: **Tergau, Enno et al**
**Patentanwälte Tergau & Pohl Postfach 11 93**
**47 Hefnersplatz 3**
**D-8500 Nürnberg 11(DE)**

(54) **Reizstromgerät.**

(57) Die Erfindung betrifft ein Reizstromgerät zur Elektrotherapie und/oder zur elektromedizinisch-diagnostischen Anwendung mit einem Funktionsgenerator zur Erzeugung von Spannungsimpulsen vorwählbarer Kurvenform, einem Operationsverstärker (OP1), dessen erster Eingang mit dem Ausgang des Funktionsgenerators verbunden ist und einer Endstufe (Leistungstransistor T3) zur Steuerung des Patientenstromkreises (4). Die Endstufe ist vom Ausgang (3) eines Multiplizierers (Zwei-Quadranten-Steilheilsmultiplizierer 1) angesteuert, dessen erster Eingang (B) von einer konstanten regelbaren Gleichspannung und dessen zweiter Eingang (2) über den Operationsverstärker (OP1) von den amplitudenkonstanten Spannungsimpulsen des Funktionsgenerators gespeist ist.

EP 0 335 266 A1

## Reizstromgerät

Die Erfindung betrifft ein Reizstromgerät mit den im Oberbegriff des Anspruches 1 angegebenen Merkmalen.

Bei der Elektrotherapie bzw. der elektromedizinischen Diagnostik wird mittels eines Reizstromgerätes über entsprechende Applikationselektroden ein Strom vorwählbarer Kurvenform und einstellbarer Maximalamplitude durch entsprechende Körperteile eines Patienten geleitet. Die Intensität des Stromes soll im wesentlichen unabhängig vom elektrischen Widerstand der behandelten Körperpartie sein.

Reizstromgeräte der eingangs genannten Art sind beispielsweise aus DE-PS 22 57 189 bekannt. Dabei wird ein Funktionsgenerator zur Erzeugung von Spannungsimpulsen vorwählbarer Kurvenform verwendet. Zwischen den nicht invertierenden Eingang eines als Differenzverstärker wirkenden Operationsverstärkers und den Funktionsgenerator ist ein Potentiometer eingeschaltet, mit dem die Maximalamplitude der Spannungsimpulse des Funktionsgenerators einstellbar ist. Der Ausgang des Differenzverstärkers steuert als Endstufe den Patientenstromkreis, in dem somit ein Strom mit der entsprechenden Kurvenform und einer regelbaren Maximalamplitude erzeugt wird. Eine an einem seriell im Patientenstromkreis liegenden Widerstand abfallende Spannung wird über einen Rückkoppelzweig auf den invertierenden Eingang des Operationsverstärkers gelegt.

Durch eine Eichung der Abgriffstellung des Potentiometers in Amplitudenwerten des Reizstromes kann die Stromintensität eingestellt und abgelesen werden. Dies ist von Nachteil, da sich das Potentiometer als Einstellelement während des Dauereinsatzes beispielsweise in seiner Charakteristik verändern kann, wodurch eine Abweichung des tatsächlichen Patientenspitzenstromes vom Sollwert auftreten kann. Darüber hinaus zeigen Potentiometer im Laufe der Zeit beispielsweise durch Staubablagerungen Verschleißerscheinungen und Unregelmäßigkeiten, die zu hohen Spannungsschwankungen während des Verstellens des Potentiometers führen können. Diese wiederum führen zu kurzzeitigen, starken Stromschwankungen im Patientenstromkreis, was beispielsweise Muskelzuckungen, Reizungen u.dgl. auslösen kann.

Der Erfindung liegt die Aufgabe zugrunde, ein Reizstromgerät der eingangs genannten Art derart zu verbessern, daß die vorstehend genannten Nachteile vermieden werden.

Die Lösung dieser Aufgabe ist in den kennzeichnenden Merkmalen des Anspruches 1 angegeben. Demnach ist die Endstufe des Reizstromgerätes vom Ausgang eines Multiplizierers angesteuert, dessen erster Eingang von einer konstanten, regelbaren Gleichspannung und dessen zweiter Eingang über den Operationsverstärker von den amplitudenkonstanten Spannungsimpulsen des Funktionsgenerators gespeist ist. Durch diesen Schaltungsaufbau ist die Erzeugung der gewünschten Kurvenform und die Einstellung der Maximalamplitude des Patientenstromes funktional getrennt. Der Funktionsgenerator stellt amplitudenkonstante Spannungsimpulse der gewünschten Kurvenform her, während der Patientenspitzenstrom über eine konstante regelbare Gleichspannung einstellbar ist. Beide Funktionen werden durch den Multiplizierer vereint, dessen Ausgang die Basis eines als Endstufe fungierenden Leistungstransistors steuert.

Eine besonders einfache, dabei jedoch zuverlässige und funktionell für die Anwendung in einem Reizstromgerät vollkommen ausreichende Bauweise des Multiplizierers ist in Anspruch 2 angegeben Bei einem Zwei-Quadranten-Steilheitsmultiplizierer kann der Ausgang Spannungen verschiedener Polarität erzeugen, wobei darauf zu achten ist, daß an einem der beiden Eingänge jeweils Spannungen mit einer bestimmten Polarität - also beispielsweise ausschließlich negative Spannungen - anliegen. Dies wird beim Erfindungsgegenstand dahingehend ausgenutzt, daß die eine bestimmte Polarität aufweisenden Spannungsimpulse des Funktionsgenerators auf den entsprechenden Eingang des Multiplizierers gelegt werden, wogegen die in Höhe und Polarität regelbare Gleichspannung auf den zweiten Eingang des Multiplizierers gelegt wird. Dessen Ausgangspolarität ist also auf besonders einfache Weise durch die Polarität der regelbaren Gleichspannung bestimmbar.

Ein besonderer Vorteil des erfindungsgemäßen Gerätes gegenüber herkömmlichen Reizstromgeräten kommt durch die Ausgestaltung nach Anspruch 3 zu tragen. Demnach dient der erste Eingang des Multiplizierers als Meßpunkt für die dem Patienten-Spitzenstrom proportionale, regelbare Gleichspannung. Es genügt also, ein einfaches Gleichspannungsmeßgerät in Einheiten des Patienten-Spitzenstromes zu eichen, um eine extrem genaue und dauerhaft exakte Ablesemöglichkeit für den Patientenspitzenstrom zu geben. Auf aufwendige, in den Patientenstromkreis eingebaute Spitzenstrommeßgeräte kann also verzichtet werden.

Da die regelbare Gleichspannung darüber hinaus in bekannter Weise durch eine moderne Gleichspannungsquelle erzeugt werden kann, die beispielsweise über eine ergonomische und pflegeleichte Folientastatur verfügt, ist das erfindungsgemäße Reizstromgerät für den Praxiseinsatz bestens geeignet. Beispielsweise kann über zwei Drucktasten die regelbare, zum Patientenspitzenstrom proportionale Gleichspannung durch Laden und Entladen eines Integrators mit vorgegebener Änderungsgeschwindigkeit schnell, komfortabel und bedienungsfreundlich eingestellt werden.

Durch die im Anspruch 4 angegebene Rückkopplung des zweiten Einganges des Operationsverstärkers kann die Temperaturdrift des als Endstufe wirkenden Leistungstransistors im Patientenstromkreis ausgeglichen werden. Damit entspricht der tatsächliche Patientenspitzenstrom extrem genau dem Sollwert. Weiterhin wird die gewünschte Kurvenform des Reizstromes sehr getreu eingehalten.

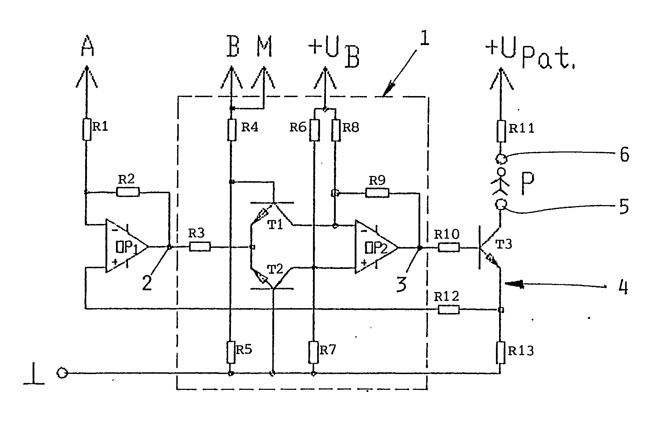

Die Erfindung wird in einem Ausführungsbeispiel anhand der beiliegenden Zeichnung näher erläutert. Letztere zeigt einen Schaltplan der Kernschaltung des erfindungsgemäßen Reizstromgerätes, wobei auf übliche Bauteile wie den Funktionsgenerator und die regelbare Gleichspannungsquelle verzichtet wurde.

Die Kernschaltung weist zwei Eingänge auf, nämlich den Impulsspannungseingang (A) und den Eingang (B) für die regelbare Gleichspannung. Der Impulsspannungseingang (A) ist mit dem Ausgang eines nicht dargestellten Funktionsgenerators verbunden, der beispielsweise Drei ecks-, Sägezahn- und/oder Rechteckimpulse einstellbarer Frequenz und positiver Polarität erzeugt. Falls ein zeitlich konstanter Patientenstrom gewünscht wird, kann der Funktionsgenerator auch eine positive Gleichspannung erzeugen. Der Impulsspannungseingang (A) ist über einen Vorschaltwiderstand (R1) mit dem invertierenden Eingang des Operationsverstärkers (OP1) verbunden, dessen Ausgang über den Rückkoppelwiderstand (R2) auf den invertierenden Eingang rückgekoppelt ist. Falls abweichend von der Zeichnung der nicht invertierende Eingang des Operationsverstärkers (OP1) auf Masse gelegt ist, so werden die am Eingang (A) eintreffenden, positiven Spannungsimpulse vom Funktionsgenerator in ihrer Polarität geändert, also negativ gemacht.

Die beiden Transistoren (T1,T2), der Eingangswiderstand (R3), die Spannungsteiler (R4,R5 bzw. R6,R7), der Widerstand (R8) sowie der über den Rückkoppelwiderstand (R9) vom Ausgang auf den invertierenden Eingang rückgekoppelte Operationsverstärker (OP2) bilden den in seinem Aufbau und seiner Wirkungsweise wohlbekannten Zwei-Quadranten-Steilheitsmultiplizierer (1) mit seinen beiden Eingängen (2,B). Am Eingang (2) liegen die invertierten, negativen Spannungsimpulse des Funktionsgenerators an, wogegen am Eingang (B) die regelbare Gleichspannung zur Einstellung der maximalen Patientenstromamplitude anliegt. Durch die Multiplikationsfunktion des Zwei-Quadranten-Multiplizierers (1) entsteht an dessen Ausgang (3) eine Spannung, die den Spannungsimpulsen des Funktionsgenerators kurvengetreu folgt, wobei die Spitzenamplituden proportional zu der am Eingang (B) anliegenden Gleichspannung sind. Der Ausgang (3) steuert über den Basiswiderstand (R10) den Leistungstransistor (T3) im Patientenstromkreis (4). Letzterer besteht aus in Serie geschaltet dem Widerstand (R11), dem Patienten (P), dessen zu behandelnde Körperpartie über Elektroden (5,6) an den Stromkreis (4) angeschlossen ist, der Kollektor-Emitterstrecke des Leistungstransistors (T3) und dem Widerstand (R13) Die Versorgungsspannung ($U_{pat}$) für den Patientenstrom fällt über die vorgenannten Bauteile gegen Masse ab.

Durch die Stromeinprägung über den Leistungstransistor (T3) folgt der dem Patientenstrom entsprechende Kollektor-Emitterstrom des Leistungstransistors (T3) streng dessen Basis-Emitterstrom. Damit fließt bei konstanten thermischen Verhältnissen am Leistungstransistor (T3) im Patientenstromkreis ein Patientenstrom, dessen Maximalamplitude unabhängig vom Patientenwiderstand ist und der dem durch die Spannungsimpulse vom Funktionsgenerator und die regelbare Gleichspannung gegebenen Sollverlauf folgt. Um Abweichungen davon aufgrund thermischer Änderungen im Leistungstransistor (T3) auszugleichen, wird die am Widerstand (R13) abfallende, dem jeweiligen Patientenstrom proportionale Spannung über den Rückkoppelwiderstand (R12) auf den nicht invertierenden Eingang des Operationsverstärkers (OP1) gelegt.

Der Eingang (B) des Multiplizierers (1) für die regelbare Gleichspannung dient als Meßpunkt (M) für diese Gleichspannung. Letztere ist proportional zur Maximalamplitude des fließenden Patientenstromes. Durch eine einfache Eichung des nicht dargestellten Spannungsmeßinstrumentes in Einheiten der Stromamplitude ist also eine einfache Ablesung des maximalen Patientenstromes ohne Verwendung aufwendiger Spitzenstrommeßgeräte möglich.

<div style="text-align:center">Bezugszeichen</div>

1 Zwei-Quadranten-Steilheitsmultiplizierer
2 Eingang
3 Ausgang
4 Patientenstromkreis
5,6 Elektroden
A Impulsspannungseingang
B Eingang für regelbare Gleichspannung
M Meßpunkt
P Patient
$U_B$ Betriebsspannung
$U_{pat}$ Versorgungsspannung für Patientenstrom

5       EP 0 335 266 A1       6

R1 Vorschaltwiderstand
R2 Rückkoppelwiderstand
R3 Eingangswiderstand

$$\left.\begin{array}{l} R4 \\ R5 \end{array}\right\} \text{Spannungsteiler}$$

$$\left.\begin{array}{l} R6 \\ R7 \end{array}\right\} \text{Spannungsteiler}$$

R8 Widerstand
R9 Rückkoppelwiderstand
R10 Basiswiderstand
R11 Widerstand
R12 Rückkoppelwiderstand
R13 Widerstand
OP1 Operationsverstärker
OP2 Operationsverstärker
T1 Transistor
T2 Transistor
T3 Leistungstransistor

## Ansprüche

1. Reizstromgerät zur Elektrotherapie und/oder zur elektromedizinisch-diagnostischen Anwendung unter Applikation eines vorzugsweise im wesentlichen vom Patientenwiderstand unabhängigen Stromes vorwählbarer Kurvenform und einstellbarer Maximalamplitude auf den Patienten (P) mit
- einem Funktionsgenerator zur Erzeugung von Spannungsimpulsen vorwählbarer Kurvenform,
- einem Operationsverstärker (OP1), dessen erster Eingang (Impulsspannungseingang A) mit dem Ausgang des Funktionsgenerators verbunden ist und
- einer Endstufe (Leistungstransistor T3) zur Steuerung des Patientenstromkreises (4),
dadurch gekennzeichnet,
daß die Endstufe (Leistungstransistor T3) vom Ausgang (3) eines Multiplizierers angesteuert ist, dessen
- erster Eingang (B) von einer konstanten, regelbaren Gleichspannung und
- zweiter Eingang (2) über den Operationsverstärker (OP1) von den amplitudenkonstanten Spannungsimpulsen des Funktionsgenerators gespeist ist.

2. Reizstromgerät nach Anspruch 1,
dadurch gekennzeichnet,
daß der Multiplizierer ein Zwei-Quadranten-Steilheitsmultiplizierer (1) ist.

3. Reizstromgerät nach Anspruch 1 oder 2,
dadurch gekennzeichnet,
daß der erste Eingang (B) des Multiplizierers (Zwei-Quadranten-Steilheitsmultiplizierer 1) als Meßpunkt (M) für die dem Patienten-Spitzenstrom proportionale, regelbare Gleichspannung dient.

4. Reizstromgerät nach einem der vorgenannten Ansprüche,
dadurch gekennzeichnet,
daß die an einem seriell im Patientenstromkreis (4) liegenden Widerstand (R13) abfallende, dem jeweiligen Patientenstrom proportionale Spannung über einen Rückkoppelwiderstand (R12) auf den zweiten Eingang des Operationsverstärkers (OP1) gelegt ist.

4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-1 589 503 (BOSCH)<br>* Seite 6, Zeile 19 - Seite 10, Zeile 5; Figuren *<br>--- | 1 | A 61 N 1/08<br>A 61 N 1/18 |
| A | EP-A-0 026 324 (SIEMENS)<br>* Seite 4, Zeile 26 - Seite 7, Zeile 8; Figuren *<br>--- | 1,4 | |
| A | FR-A-2 500 689 (FAIVELEY)<br>* Insgesamt *<br>----- | | |

| RECHERCHIERTE SACHGEBIETE (Int. Cl.4) |
|---|
| A 61 N |

**Der vorliegende Recherchenbericht wurde für alle Patentansprüche erstellt**

| Recherchenort | Abschlußdatum der Recherche | Prüfer |
|---|---|---|
| DEN HAAG | 22-06-1989 | LEMERCIER D.L.L. |